# EUROPÄISCHE PATENTSCHRIFT

(11) **EP 2 235 194 B1**
(45) Veröffentlichungstag und Bekanntmachung des Hinweises auf die Patenterteilung: **20.07.2011**
(21) Anmeldenummer: 09704118.0
(22) Anmeldetag: 23.01.2009
(51) Int. Cl.: C12P 13/00

(54) **Verfahren zur fermentativen Herstellung von 1,5-Diaminopentan**
Method for fermentatively producing 1,5-diaminopentane
Procédé de production fermentative de 1,5-diaminopentane

(30) Priorität: 23.01.2008 EP 08150575
(43) Veröffentlichungstag der Anmeldung: 06.10.2010
(73) Patentinhaber: BASF SE, 67056 Ludwigshafen (DE)
(72) Erfinder: VÖLKERT, Martin, 67063 Ludwigshafen (DE); ZELDER, Oskar, 67346 Speyer (DE); ERNST, Burkhard, 68199 Mannheim (DE); JEONG, Weol Kyu, Jeollabuk-do 573-879 (KR)
(86) Internationale Anmeldenummer: PCT/EP2009/050778
(87) Internationale Veröffentlichungsnummer: WO 2009/092793

(56) Entgegenhaltungen:
- EP-A- 1 482 055
- WO-A-2007/113127
- DATABASE WPI Week 200459 Thomson Scientific, London, GB; AN 2004-606664 XP002535219 & JP 2004 222569 A (TORAY IND INC) 12. August 2004 (2004-08-12)

## Beschreibung

Die vorliegende Erfindung betrifft ein Verfahren zur Isolierung von 1,5-Diaminopentan (DAP) aus DAP-haltigen Fermentationsbrühen, ein Verfahren zur fermentativen Herstellung von DAP unter Verwendung dieser Isolierungsmethode sowie ein Verfahren zur Herstellung von DAP-haltigen Polymeren unter Einsatz des in dieser Weise isolierten bzw. fermentativ hergestellten DAPs.

### HINTERGRUND DER ERFINDUNG

1,5-Diaminopentan (häufig auch bezeichnet als Pentamethylendiamin oder Cadaverin; im Folgenden bezeichnet als DAP) stellt einen wichtigen Grundstoff der chemischen Industrie dar. Beispielsweise findet DAP Verwendung bei der Herstellung von Polyamiden, Polyharnstoffen oder Polyurethanen sowie von Copolymeren daraus.

Zudem ist die fermentative oder enzymatische Herstellung von DAP durch Decarboxylierung von Lysin seit längerem bekannt. Dabei werden verschiedene Verfahren zur Isolierung des Wertproduktes aus der Fermentationsbrühe beschrieben.

So wird beispielsweise in der EP-A-1 482 055 die enzymatische Decarboxylierung von Lysin in Gegenwart einer Dicarbonsäure zur Einstellung des pH-Wertes während der Umsetzung beschrieben. Das bei der Herstellung anfallende DAP-Dicarboxylat wird isoliert, indem man die wertstoffhaltige Lösung zunächst mit Aktivkohle entfärbt, aufkonzentriert und DAP-Dicarboxylat durch eine Kühlungskristallisation auskristallisiert.

In der WO-A-2006/123778 ist die Herstellung von DAP-Carbonat durch enzymatische Decarboxylierung von Lysin in Gegenwart von Kohlendioxid beschrieben. Durch Aufkonzentrierung der Reaktionslösung und Abspaltung von Kohlendioxid wird DAP gebildet.

Die JP 2004-208 646 beschreibt die Herstellung von DAP-Dicarboxylat durch enzymatische Decarboxylierung einer L-Lysin-Dicarboxylat enthaltenden Lösung und Ausfällung von DAP-Dicarboxylat durch Zugabe eines organischen Lösungsmittels, ausgewählt unter Alkoholen, Ketonen und Nitrilen.

Die JP 2004-222 569 beschreibt die Herstellung von DAP unter Verwendung eines L-Lysin-Decarboxylase-exprimierenden coryneformen Bakteriums, Einstellung des Kulturüberstandes auf pH 12 und Extraktion von DAP mit einem polaren organischen Lösungsmittel.

Schließlich beschreibt die JP 2004-000 114 die Herstellung von DAP durch Umsetzung von hochkonzentriertem L-Lysin-Monohydrochlorid mit L-Lysin-Decarboxylaseexprimierenden E. coli-Zellen, Einstellung der Reaktionslösung auf pH ≥13 und Extraktion des Reaktionsproduktes mit einem polaren organischen Lösungsmittel und anschließender Destillation.

Insbesondere die aus dem Stand der Technik bekannten, auf einer Extraktion von DAP mit Hilfe eines organischen Lösungsmittels basierenden Verfahren sind jedoch mit dem Nachteil behaftet, dass die Wertstoffausbeute nicht optimal ist und insbesondere der Extraktionsschritt zu langsam verläuft und das Gesamtverfahren daher zu zeitaufwändig ist, was für eine Anwendung der Herstellung im technischen Maßstab von großem Nachteil ist.

### ZUSAMMENFASSUNG DER ERFINDUNG

Der vorliegenden Erfindung liegt somit die Aufgabe zugrunde, die Isolierung von DAP (Cadaverin) aus Fermentationsbrühen weiter zu verbessern. Insbesondere sollte die Ausbeute an Wertstoff weiter erhöht und der erforderliche Zeitaufwand für die Isolierung, insbesondere die Lösungsmittel-basierte Extraktion, verbessert werden.

Überraschenderweise wurde diese Aufgabe durch Bereitstellung eines Verfahrens gelöst, bei welchem man die auf einen alkalischen pH-Wert eingestellte Fermentationsbrühe thermisch behandelt und anschließend mit einem geeigneten organischen Extraktionsmittel extrahiert. Dabei wurde überraschenderweise festgestellt, dass DAPhaltige Nebenprodukte der Fermentation, insbesondere Acetyl-DAP unter Freisetzung des Wertproduktes hydrolytisch gespalten werden und überraschenderweise außerdem die Geschwindigkeit der Phasentrennung während des Extraktionsschrittes deutlich erhöht werden kann. Die erhöhte Geschwindigkeit der Phasentrennung ist besonders evident bei der Aufarbeitung von Fermentationsbrühen aus der Fermentation von Mikroorganismen in Gegenwart von komplexen Nährmedien, wie z.B. Hefeextrakt.

### FIGURENBESCHREIBUNG

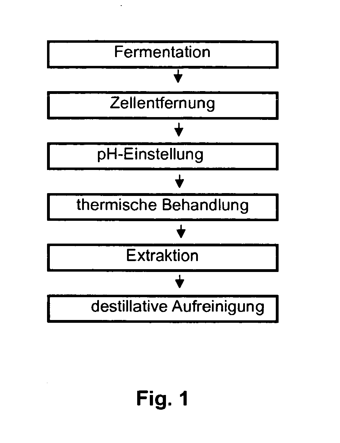

Figur 1 zeigt ein Blockschema für den Verlauf einer speziellen erfindungsgemäßen Ausführungsform eines Gesamtprozesses zur Isolierung von DAP aus einer Fermentationsbrühe.
Figur 2 veranschaulicht die hydrolytische Spaltung von Acetyl-DAP (Dreiecke) unter Bildung von DAP (Rauten) im Verlauf einer fünfstündigen thermischen Behandlung einer Fermentationsbrühe durch Rückflusskochen bei pH 13,7. Der Gehalt an restlichem Lysin (Quadrate) bleibt während der thermischen Behandlung unverändert.
Figur 3 veranschaulicht die Freisetzung von Ammoniak während des Aufheizvorgangs und des anschließenden Rückflusskochens der Fermentationsbrühe. Untere Kurve - Gasmenge; obere Kurve - Innentemperaturverlauf.

### DETAILLIERTE BESCHREIBUNG DER ERFINDUNG

### 1. Bevorzugte Ausführungsformen

Gegenstand der Erfindung ist ein Verfahren zur Isolierung von 1,5-Diaminopentan (DAP) aus einer DAP-haltigen Fermentationsbrühe, wobei man die Fermentationsbrühe a) alkalisiert, b) thermisch behandelt, c) DAP mit einem organischen Extraktionsmittel extrahiert, und d) DAP aus der abgetrennten organischen Phase isoliert, wie im beiliegenden Hamptanspruch näher definiert.

In einer ersten speziellen Ausgestaltung des Verfahrens wird die Fermentationsbrühe auf einen pH-Wert von ≥11 oder ≥12, wie insbesondere ≥12 bis 14, oder 12,5 bis 13,8, oder 13 bis 13,8, oder 13,5 bis 13,7 eingestellt. Dazu erfolgt die pH-Wert-Einstellung insbesondere durch Zugabe eines Alkali- oder Erdalkalimetallhydroxids, wie eines Na-, K- oder Ca-Hydroxids.

Die Stoffverteilung kann durch Anpassung des pH Wertes weiter optimiert werden, wobei bei pH-Werten oberhalb von etwa 12,5 optimale Stoffübergangsbedingungen eingestellt können.

Ebenso kann die Spaltung von gegebenenfalls enthaltenem Acetyl-DAP durch Anpassung des pH-Wertes weiter optimiert werden, wobei - abhängig von der Menge an enthaltenem Acetyl-DAP - bei pH-Werten oberhalb von etwa 13 optimale Spaltungsbedingungen (Spaltungskinetiken) eingestellt werden können.

Gegebenenfalls können vor der Alkalisierung aus der Fermentationsbrühe zelluläre Bestandteile entfernt werden. Methoden zur Entfernung der zellulären Bestandteile sind dem Fachmann geläufig (z.B. Separatoren, Dekanter, Flockung, Filtrationsverfahren, bzw. Kombinationen mehrerer solcher Prozessschritte).

In einer weiteren Ausgestaltung des Verfahrens wird die alkalisierte Fermentationsbrühe thermisch behandelt, indem man, z.B. Batch-weise oder kontinuierlich, auf Rückflusstemperatur, wie z.B. 90-110 °C bei Normaldruck oder auf eine höhere Temperatur bei Überdruck, wie z.B. 0-100 bar, insbesondere 0-25 bar Überdruck, erhitzt. Dabei wird die thermische Behandlung unter Bedingungen durchgeführt, die eine, bevorzugt im wesentlichen quantitative, hydrolytische Spaltung von gegebenenfalls vorhandenem Acetyl-DAP bewirken. Der Fachmann kann dazu die wesentlichen Verfahrensparameter, wie Druck, Temperatur und Verweilzeit dem Erfordernis entsprechend aufeinander abstimmen. Der Begriff "Acetyl-DAP" umfasst Mono- und Di-Acetyl-DAP, wobei gewöhnlich aber überwiegend die Mono-Acetylform vorliegt. In einer weiteren Ausführungsform kann diese Erhitzung mehrstufig durchgeführt werden, z.B. auch unter Wiedergewinnung des freigesetzten Ammoniaks durch Zwischenentspannung.

In einer weiteren Ausgestaltung des erfindungsgemäßen Verfahrens wird die DAP-Extraktion mit einem organischen Lösungsmittel mit Mischungslücke mit Wasser, das möglichst polar und im Alkalischen stabil ist, wie insbesondere einem polaren, insbesondere dipolar-protischen, organischen Lösungsmittel durchgeführt. Geeignete Lösungsmittel werden in einem folgenden Abschnitt beschrieben

In einer bevorzugten Ausführungsform wird die DAP-Extraktion und/oder die anschließende Phasentrennung diskontinuierlich bei erhöhter Temperatur durchgeführt. Weitere Ausgestaltungen der Extraktion und der Aufarbeitung des DAP-haltigen Extraktes werden in einem folgenden Abschnitt beschrieben.

Insbesondere eignet sich das erfindungsgemäße Verfahren für die Aufarbeitung von Fermentationsbrühen aus der Fermentation eines Mikroorganismus in einem komplexen, wie z.B. Hefeextrakt-haltigen, Kulturmedium.

Ein weiterer Gegenstand der Erfindung ist ein Verfahren zur fermentativen Herstellung von DAP, wobei man einen Lysin-produzierenden Mikroorganismus unter Lysin- und gegebenenfalls DAP-produzierenden Bedingungen kultiviert und das gebildete DAP unter Anwendung eines DAP-Isolationsverfahrens nach obiger Definition isoliert.

Insbesondere kann dabei die Fermentation in einem Kulturmedium mit komplexen Medienbestandteilen durchgeführt werden. Dabei kann man einen Lysin-produzierenden Mikroorganismus verwenden, der zusätzlich Lysin Decarboxylase-Aktivität, wie z.B. eine heterologe, d.h. aus einem anderen Organismus stammende, Lysin Decarboxylase (LDC), exprimiert.

"Komplexe Nähr- oder Kulturmedien" oder "Komplexmedien" sind erfindungsgemäß an sich bekannte Medien, welche komplex zusammengesetzte Stoffgemische umfassen, wie z.B. Maisquellwasser, Trypton, Bacton, Sojahydrolysat und insbesondere Hefeextrakt.

Andererseits kann auch eine Lysin-haltige Fermentationsbrühe mit gereinigter, gegebenenfalls immobilisierter Lysin-Decarboxylase in Kontakt gebracht werden, um Lysin zu DAP zu decarboxylieren oder ein weiterer, gegebenenfalls immobilisierter LDCexprimierender Mikroorganismus kann der Brühe zugesetzt oder diese damit in Kontakt gebracht werden. Geeignete Verfahren sind dabei im Stand der Technik beschrieben, worauf hiermit ausdrücklich Bezug genommen wird. (vgl. z.B. JP 2002-223771).

Grundsätzlich kann dabei das gesamte oben beschriebene Isolierungsverfahren bzw. das oben beschriebene fermentative Herstellungsverfahren oder einzelne Schritte davon kontinuierlich oder diskontinuierlich, in Batch- oder Semibatch-, oder Fed-Batch- oder repeated (Fed-)Batch-Fahrweise, erfolgen.

Ein weiterer Gegenstand der Erfindung betrifft ein Verfahren zur Herstellung eines DAP-haltigen Polymers, wobei man zunächst monomeres DAP nach einem Verfahren gemäß obiger Definition fermentativ herstellt und isoliert und zusammen mit wenigsten einem weiteren Comonomer polymerisiert. Dabei kann das Comonomer insbesondere ausgewählt sein unter Polycarbonsäuren, wie insbesondere Dicarbonsäuren mit 4 bis 12 Kohlenstoffatomen, deren Estern und Anhydriden; sowie Polyisocyanaten, wie insbesondere Diisocyanaten mit einer C₂-C₁₀-Alkylen-Brückengruppe oder cyclischen Brückengruppen. Nichtlimitierende Beispiele geeigneter Dicarbonsäuren sind Bernsteinsäure, Glutarsäure, Adipinsäure, Pimelinsäure, Suberinsäure, Azelainsäure, Sebacinsäure usw. Nichtlimitierende Beispiele geeigneter Diisocyanate sind Methylendiphenyldiisocyanat (MDI), Toluoldiisocyanat (TDI), Hexamethylendiisocyanat (HDI) und Isophorondiisocyanat. Dabei werden insbesondere Polymere vom Polyamid-, Polyharnstoff- oder Polyurethan-Typ gebildet, wie z.B. Polyamid 5,10 oder Polyamid 5,6.

Für das Polymerisationsverfahren setzt man wenigstens ein Comonomer dem isolierten DAP zu oder setzt ein Gemisch aus DAP und wenigstens einem Comonomer aus einer DAP-Fällung ein. Beispielsweise kann ein geeignetes DAP/Comonomer-Gemisch aus einer oben beschriebenen Salzfällung von DAP aus einem destillativ aufgearbeiteten DAP-Extrakt resultieren. Das Comonomer ist dabei vorzugsweise eine Polycarbonsäure, wie beispielsweise Sebacinsäure.

### 2. Allgemeine Angaben zur fermentativen Lysin- oder DAP-Herstellung

### 2.1 Mikroorganismen

Die vorliegende Erfindung ist grundsätzlich auf die Aufarbeitung jeglicher DAP-haltiger Fermentationsbrühen anwendbar. Auch bezüglich der bei der Fermentation verwendeten Mikroorganismen bestehen grundsätzlich keinerlei Beschränkungen. Dabei kann es sich um natürlich vorkommende, um durch Mutation und Selektion verbesserte, insbesondere aber um rekombinant hergestellte Mikroorganismen, wie Pilze, insbesondere aber Bakterien handeln. Diese Mikroorganismen besitzen entweder direkt die Fähigkeit zur Produktion von DAP und/oder DAP-Derivaten, wie Acetyl-DAP, zumindest aber sind sie zur fermentativen Herstellung von Lysin, insbesondere L-Lysin befähigt. Insbesondere ist ein verwendetes rekombinantes Bakterium zur Lysin-Biosynthese über den Diaminopimelat-Weg ("DAP-Weg"), den Succinylase-Weg oder den Dehydrogenase-Weg befähigt.

Diese Mikroorganismen können Lysin, insbesondere L-Lysin, aus Glucose, Saccharose, Lactose, Fructose, Maltose, Melasse, Stärke, Cellulose oder aus Glycerin, Fettsäuren oder Pflanzenölen oder Ethanol produzieren und vorzugsweise das gebildete Lysin zumindest teilweise in den extrazellulären Raum abgeben. Vorzugsweise sind dies coryneforme Bakterien, insbesondere der Gattung Corynebacterium oder der Gattung Brevibacterium. Aus der Gattung *Corynebacterium* ist insbesondere die Art *Corynebacterium glutamicum* zu nennen, die in der Fachwelt für ihre Fähigkeit bekannt ist, L-Aminosäuren zu produzieren.

Als Beispiele für geeignete Stämme coryneformer Bakterien sind solche der Gattung *Corynebacterium,* insbesondere der Art *Corynebacterium glutamicum (C. glutamicum),* wie
*Corynebacterium glutamicum* ATCC 13032,
*Corynebacterium acetoglutamicum* ATCC 15806,
*Corynebacterium acetoacidophilum* ATCC 13870,
*Corynebacterium thermoaminogenes* FERM BP-1539,
*Corynebacterium melassecola* ATCC 17965
   oder
der Gattung *Brevibacterium,* wie
*Brevibacterium flavum* ATCC 14067
*Brevibacterium lactofermentum* ATCC 13869 und
*Brevibacterium divaricatum* ATCC 14020 zu nennen;
   oder davon abgeleitete Stämme, wie
*Corynebacterium glutamicum* KFCC10065
*Corynebacterium glutamicum* ATCC21608

Mit der Abkürzung KFCC ist die Korean Federation of Culture Collection gemeint, mit der Abkürzung ATCC die American type strain culture collection, mit der Abkürzung FERM BP die Sammlung des National institute of Bioscience and Human-Technology, Agency of Industrial Science and Technology, Japan bezeichnet.

### 2.2 Durchführung der Fermentation

Erfindungsgemäß aufzuarbeitende Fermentationsbrühen stammen beispielsweise aus der Kultivierung von rekombinanten coryneformen Bakterien, welche über einen die Lysin-Biosynthese fördernden, wenigstens ein Lysinbiosynthese-Gen betreffenden deregulierenden Eingriff, vermehrt Lysin, insbesondere L-Lysin, oder ein Lysin-haltiges Stoffgemisch produzieren und / oder welche zusätzlich ein Enzym mit Lysin Decarboxylase-Aktivität überexprimieren und DAP und/oder Acetyl-DAP akkumulieren. Letztere sind damit zur direkten DAP-Produktion befähigt.

An der Lysin-Biosynthese beteiligte Gene und ein zugeordneter, die Lysin-Biosynthese fördernder deregulativer Eingriff sind in folgender Tabelle1 zusammengefasst.

**Tabelle 1: Beispiele deregulierbarer Gene und Genprodukte**

| **Enzym (Genprodukt)** | *Gen* | Deregulation |
|---|---|---|
| Aspartokinase | *ask* | Aufhebung der Feedback Inhibition durch Punktmutation (Eggeling et al., (eds.), Handbook of Corynebac- terium glutamicum, pages 20.2.2 (CRC press, 2005)) und Amplifikation) |
| Aspartatesemialdehyde dehydrogenase | *asd* | Amplifikation |
| Dihydrodipicolinate synthase | *dapA* | Amplifikation |
| Dihydrodipicolinate reductase | *dapB* | Amplifikation |
| Tetrahydrodipicolinate succinylase | *dapD* | Amplifikation |
| Succinyl-amino-ketopimelate transaminase | *dapC* | Amplifikation |
| Succinyl-diamino-pimelate desuccinylase | *dapE* | Amplifikation |
| Diaminopimelate dehydrogenase | *ddh* | Amplifikation |
| Diaminopimelate epimerase | *dapF* | Amplifikation |
| Arginyl-tRNA synthetase | *argS* | Amplifikation |
| Diaminopimelate decarboxylase | *lysA* | Amplifikation |
| Pyruvate carboxylase | *pycA* | Aufhebung der Feedback Inhibition durch Punktmutation (EP1108790) und Amplifikation |
| Phosphoenolpyruvate carboxylase | ppc | Amplifikation |
| Glucose-6-phosphate dehydrogenase | *zwf* | Aufhebung der Feedback Inhibition durch Punktmutation (US2003/0175911) und Amplifikation |
| Transketolase | *tkt* | Amplifikation |
| Transaldolase | *tal* | Amplifikation |
| 6-Phosphogluconolactonase | *pgl* | Amplifikation |
| Fructose 1,6-biphosphatase | *fbp* | Amplifikation |
| Homoserine dehydrogenase | *hom* | Attenuierung durch Punktmutation (EP1108790) |
| Phophoenolpyruvate carboxykinase | *pck* | Knock-out oder Silencing durch Mutation u.a. |
| Succinyl-CoA synthetase | *sucC* | Attenuierung durch Punktmutation (WO 05/58945) |
| Methylmalonyl-CoA mutase | | Attenuierung durch Punktmutation (WO 05/58945) |

Ein Verfahren zur Herstellung von DAP unter Verwendung rekombinanter Mikroorganismen mit dereguliertem Lysin-Decarboxylasegen und wenigstens einem weiteren deregulierten, z.B. an der Lysinbiosynthese beteiligten Gen, ist aus der WO 2007/113127 bekannt.

Wie ersichtlich, ist eine "Deregulation" im weitesten Sinne zu verstehen und umfasst sowohl eine Erhöhung oder Verringerung oder Abschaltung einer Enzymaktivität in verschiedenster Weise, z.B. durch Erhöhung oder Verringerung der Kopienzahl von Enzymmolekülen im Mikroorganismus oder eine Veränderung einer anderen, die Lysin-Biosynthese verringernden Eigenschaft.

Das Enzym Lysin Decarboxylase (E.C. 4.1.1.18.) katalysiert die Decarboxylierung von L-Lysin zu DAP. Das Enzym ist beispielsweise das *cadA* Genprodukt (Kyoto Encyclopedia of Genes and Genomes, Entry b4131) oder das *IdcC* Genprodukt (Kyoto Encyclopedia of Genes and Genomes, Entry JW0181). Deren Verwendung zur Herstellung rekombinanter Mikroorganismen für die Cadaverin-Produktion sind dem Fachmann bekannt (vgl. z.B. EP-A-1 482 055).

Zur Erzielung einer Überexpression/Deregulierung kann der Fachmann unterschiedliche Maßnahmen einzeln oder in Kombination ergreifen. So kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Lysin-Produktion zu steigern. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in einem oder mehreren Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Biotechnology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0472869, im US Patent 4,601,893, bei Schwarzer und Pühler (Biotechnology 9, 84-87 (1991), bei Remscheid et al. (Applied and Environmental Microbiology 60,126-132 (1994), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58,.191-195 (1998)), bei Makrides (Microbiological Reviews 60 : 512-538 (1996) und in bekannten Lehrbüchern der Genetik und Molekularbiologie.

Zur Herstellung geeigneter Produktionsstämme werden Expressionskonstrukte oder Vektoren verwendet, enthaltend unter der genetischen Kontrolle regulativer Nukleinsäuresequenzen eine für eine gewünschte Enzymaktivität kodierende Nukleinsäuresequenz. Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Aktivierungssequenzen sowie Enhancer und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: die Promotoren, ddh, amy, lysc, dapA, lysA aus Corynebacterium glutamicum, aber auch gram-positiven Promotoren SPO2 wie sie in Bacillus Subtilis and Its Closest Relatives, Sonenshein, Abraham L.,Hoch, James A., Losick, Richard; ASM Press, District of Columbia, Washington und Patek M. Eikmanns BJ. Patek J. Sahm H. Microbiology. 142 1297-309, 1996 beschrieben sind, oder aber auch cos-, tac-, trp-, tet-, trp-tet-, Ipp-, lac-, Ipp-lac-, laclq-, T7-, T5-, T3-, gal-, trc-, ara-, SP6-, λ-PR- oder im λ-PL-Promotor, die vorteilhafterweise in gram-negativen Bakterien Anwendung finden. Bevorzugt ist auch die Verwendung induzierbarer Promotoren, wie z.B. licht- und insbesondere temperaturinduzierbarer Promotoren, wie der PᵣPₗ-Promotor. Prinzipiell können alle natürlichen Promotoren mit ihren Regulationssequenzen verwendet werden. Darüber hinaus können auch synthetische Promotoren, wie Mehrfachpromotoren (vgl. z.B. WO2006/069711) vorteilhaft verwendet werden.

Die genannten regulatorischen Sequenzen sollen die gezielte Expression der Nukleinsäuresequenzen ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors, einer geeigneten Shine-Dalgarnow-Sequenz mit einer Lysinbiosynthese-Nukleotidsequenz sowie einem geeigneten Terminationssignal. Dazu verwendet man gängige Rekombinations- und Klonierungstechniken, wie sie beispielsweise in Current Protocols in Molecular Biology, 1993, John Wiley & Sons, Incorporated, New York New York, PCR Methods, Gelfand, David H., Innis, Michael A., Sninsky, John J. 1999, Academic Press, Incorporated, California, San Diego, ., PCR Cloning Protocols, Methods in Molecular Biology Ser., Vol. 192, 2nd ed., Humana Press, New Jersey, Totowa, T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Plasmide eignen sich solche, die in coryneformen Bakterien repliziert werden. Zahlreiche bekannte Plasmidvektoren, wie z. B. pZ1 (Menkel et al., Applied and Environmental Microbiology (1989) 64: 549-554), pEKEx1 (Eikmanns et al., Gene 102: 93-98 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie z. B. pCLiK5MCS, oder solche, die auf pCG4 (US-A 4,489,160) oder pNG2 (Serwold-Davis et al., FEMS Microbiology Letters 66, 119-124 (1990)) oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Remscheid et al. (Applied and Environmental Microbiology 60,126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Sirnon et al., Bio/ Technology 1,784-791 (1983)), pK18mob oder pK19mob (Schäfer et al., Gene 145,69-73 (1994)), Bernard et al., Journal of Molecular Biology, 234: 534-541 (1993)), pEM1 (Schrumpf et al. 1991, Journal of Bacteriology 173: 4510-4516) oder pBGS8 (Spratt et al., 1986, Gene 41: 337-342) in Frage. Der Plasmidvektor, der das zu amplifizierende Gen enthält, wird anschließend durch Transformation in den gewünschten Stamm von C. glutamicum überführt. Methoden zur Transformation sind beispielsweise bei Thierbach et al. (Applied Microbiology and Biotechnology 29, 356-362 (1988)), Dunican und Shivnan (Biotechnology 7, 1067-1070 (1989)) und Tauch et al. (FEMS Microbiological Letters 123,343-347 (1994)) beschrieben.

Enzyme können durch Mutationen in den korrespondierenden Genen derart in ihrer Aktivität beeinflusst werden, dass es zu einer teilweisen oder vollständigen Verringerung der Reaktionsgeschwindigkeit der enzymatischen Reaktion kommt. Beispiele für solche Mutationen sind dem Fachmann bekannt (Motoyama H. Yano H. Terasaki Y. Anazawa H. Applied & Environmental Microbiology. 67:3064-70, 2001, Eikmanns BJ. Eggeling L. Sahm H. Antonie van Leeuwenhoek. 64:145-63, 1993-94.). Mit dieser Maßnahme können z.B. mit der erfindungsgemäßen Lysinbiosynthese konkurrierende Reaktionen ausgeschaltet oder verlangsamt werden. (Nakayama: "Breeding of Amino Acid Producing Microorganisms", in: Overproduction of Microbial Products, Krumphanzl, Sikyta, Vanek (eds.), Academic Press, London, UK, 1982).

Zusätzlich kann es für die Produktion von L-Lysin vorteilhaft sein, neben einer Expression bzw. Verstärkung der Lysinbiosynthese-Gene eines oder mehrere Enzyme vorgeschalteter Biosyntheseweges, wie z.B. des Pentose-Phosphat-Stoffwechsels, des Zitronensäure-Zyklus, oder des Aminosäure-Exports zu verstärken.

Die erfindungsgemäß verwendeten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) zur Produktion von L-Lysin, kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehreren Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl, Sonnenblumenöl, Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas bzw. Ammoniakwasser oder Ammoniumsalze, wie Ammoniumsulfat, Ammoniumchlorid, Ammoniumphosphat, Ammoniumcarbonat, Ammoniumcarbamat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphat-, Carbonat- oder Sulfatsalze von Calcium, Magnesium, Natrium, Kobalt, Molybdän, Kalium, Mangan, Zink, Kupfer und Eisen sowie Borsäure.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Kulturmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (z.B. 20 min bei 1 bar Überdruck (2 bar absolut) und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder portionsweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Fermentation lässt sich während der Fermentation durch Zugabe von basischen Verbindungen wie z.B. Natriumhydroxid, Kaliumhydroxid, Natriumhydrogencarbonat, Ammoniak bzw. Ammoniakwasser oder saure Verbindungen wie Phosphorsäure, Salzsäure oder Schwefelsäure kontrollieren. Zur Kontrolle der Schaumentwicklung können Antischaummittel wie z. B. Fettsäurepolyglykolester, Polyalkylenglykole, Silikone oder andere eingesetzt werden (siehe z.B. Biotechnol. Progr. 2007, 23, 767-784). Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoffhaltige Gasmischungen, wie z. B. Umgebungsluft, in die Kultur eingetragen. Die Temperatur der Kultur liegt normalerweise bei 20°C bis 45°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere L-Lysin oder DAP enthaltenden, Fermentationsbrühen haben üblicherweise eine Trockenmasse von 3 bis 20 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf ≥ 0 bis 3 g/l gehalten, beziehungsweise abgesenkt wird.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren, Flockung oder eine Kombination dieser Methoden aus der Fermentationsbrühe entfernt oder vollständig in ihr belassen werden. Bevorzugt ist eine Abtrennung der Biomasse.

### 2.3 Aufarbeitung der DAP-haltigen Fermentationsbrühe

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Sofern erforderlich, können eventuell durch die Aufkonzentrierung ausgefallene Salze beispielsweise durch Filtration oder Zentrifugation abgetrennt werden. Diese aufkonzentrierte Fermentationsbrühe kann anschließend in der erfindungsgemäßen Weise aufgearbeitet werden, um DAP zu erhalten. Für die Aufarbeitung im Sinne der vorliegenden Erfindung ist eine solche Aufkonzentrierung möglich, aber nicht zwingend erforderlich.

Erfindungsgemäß wird DAP mit Hilfe eines organischen Extraktionsmittels aus der Fermentationsbrühe extrahiert. Dabei verwendet man insbesondere ein organisches Lösungsmittel mit Mischungslücke mit Wasser, das möglichst polar und im Alkalischen stabil ist, wie insbesondere ein polares, insbesondere dipolar-protisches, organisches Lösungsmittel. Geeignete Lösungsmittel sind insbesondere zyklische oder offenkettige, gegebenenfalls verzweigte Alkanole mit 3 bis 8 C-Atomen, wie insbesondere, n- und iso-Propanol, n-, sec- und iso-Butanol, oder Cyclohexanol, sowie n-Pentanol, n-Hexanol- n-Heptanol, n-Oktanol, 2-Oktanol und die ein- oder mehrfach verzweigten isomeren Formen davon. n-Butanol ist dabei besonders zu erwähnen.

In einer bevorzugten Ausführungsform wird die Extraktion und/oder die anschließende Phasentrennung diskontinuierlich bei erhöhter Temperatur durchgeführt, wobei die Temperatur durch die Siedepunkte von Wasser und des Extraktionsmittels bzw. sich möglicherweise bildender Azeotrope begrenzt ist. Mit n-Butanol als Extraktionsmittel könnten Extraktion und Phasentrennung z.B. bei etwa 25-90 °C oder bevorzugt bei 40-70 °C durchgeführt werden. Für die Extraktion werden die beiden Phasen gerührt, bis sich das Verteilungsgleichgewicht eingestellt hat, z.B. über einen Zeitraum von 10 Sekunden bis 2 Stunden, bevorzugt 5 bis 15Min. Anschließend lässt man die Phasen absitzen, bis sich die Phasen vollständig getrennt haben; dies erfolgt vorzugsweise über einen Zeitraum von 10 Sekunden bis 5 Stunden, wie z.B. 15 bis 120 oder 30 bis 90 Minuten, insbesondere auch bei einer Temperatur im Bereich von etwa 25-90 °C oder 40-70 °C im Fall von n-Butanol.

In weiteren bevorzugten Ausführungsformen wird die Extraktion des DAPs aus der Fermentationsbrühe kontinuierlich mehrstufig (z.B. in Mixer-Settler-Kombinationen) oder kontinuierlich in einer Extraktionskolonne durchgeführt.

Die apparative Ausgestaltung der erfindungsgemäß einsetzbaren Extraktionskolonnen können vom Fachmann für die jeweils zu trennenden Phasen im Rahmen routinemäßigen Optimierungsarbeiten festgelegt werden. Geeignet sind grundsätzlich Extraktionskolonnen ohne Leistungseintrag oder Extraktionskolonnen mit Leistungseintrag, wie z. B. pulsierte Kolonnen oder Kolonnen mit rotierenden Einbauten. Der Fachmann kann auch im Rahmen routinemäßiger Arbeiten Art und Materialen von Einbauten, wie Siebböden, und Kolonnen-Füllkörper, zur Optimierung der Phasentrennung in geeigneter Weise auswählen. Die theoretischen Grundlagen der Flüssig-Flüssig-Extraktion kleiner Moleküle sind allgemein bekannt (vgl. z.B. H.-J. Rehm and G. Reed, Eds., (1993), Biotechology, Volume 3 Bioprocessing, Chapter 21, VCH, Weinheim). Die Ausgestaltung von industriell anwendbaren Extraktionskolonnen ist beispielsweise beschrieben in Lo et al., Eds., (1983) Handbook of Solvent Extraction, JohnWiley& Sons, New York.

Nach der Phasentrennung erfolgt in an sich bekannter Weise die Isolierung und Aufreinigung des DAPs aus der DAP-haltigen Extraktphase. Mögliche Maßnahmen zur DAP-Gewinnung sind, ohne darauf beschränkt zu sein, insbesondere die Destillation, die Fällung als Salz mit geeigneten organischen oder anorganischen Säuren, oder Kombinationen solcher geeigneter Maßnahmen.

Die Destillation kann dabei kontinuierlich oder satzweise (diskontinuierlich) erfolgen. Es können eine einzelne oder mehrere miteinander gekoppelte Destillationskolonnen verwendet werden. Die apparative Ausgestaltung der Destillationskolonne und die Festlegung der Betriebsparameter obliegt dem Fachmann. Die jeweils eingesetzten Destillationskolonnen können dabei in an sich bekannter Weisen realisiert werden (siehe z. B. Sattler, Thermische Trennverfahren, 2. Auflage 1995, Weinheim, S. 135ff; Perry's Chemical Engineers Handbook, 7. Auflage 1997, New York, Section 13). So können die eingesetzten Destillationskolonnen z.B. trennwirksame Einbauten enthalten, wie Trennböden, z. B. Lochböden, Glockenböden oder Ventilböden, geordnete Packungen, z. B. Blech- oder Gewebepackungen, oder regellose Schüttungen von Füllkörpern. Die in der/den eingesetzten Kolonne(n) notwendige Stufenzahl und das Rücklaufverhältnis richten sich im Wesentlichen nach den Reinheitsanforderungen und der relativen Siedelage der zu trennenden Flüssigkeiten, wobei der Fachmann die konkreten Auslegungs- und Betriebsdaten nach bekannten Methoden ermitteln kann.

Die Fällung als Salz kann durch Zugabe geeigneter organischer oder anorganischer Säuren herbeigeführt werden, wie zum Beispiel Schwefelsäure, Salzsäure, Phosphorsäure, Essigsäure, Ameisensäure, Kohlensäure, Oxalsäure, usw. In einer weiteren bevorzugten Ausführungsform wird eine organische Dicarbonsäure verwendet, die zur Bildung eines Salzes führt, welches direkt oder nach Aufreinigung, z.B. durch Umkristallisation, in einer nachfolgenden Polykondensation zum Polyamid eingesetzt werden kann. Solche Dicarbonsäuren können insbesondere C₄-C₁₂-Dicarbonsäuren sein.

Die bei der Extraktion angefallene organische DAP-Phase kann auch chromatographisch aufgearbeitet werden. Für die Chromatographie wird die DAP-Phase auf ein geeignetes Harz aufgetragen, z.B. einen stark oder schwach sauren Ionentauscher, (etwa Lewatit 1468 S, Dowex Marathon C, Amberlyst 119 Wet oder andere), wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei geeigneter Temperatur aufbewahrt werden.

Die Identität und Reinheit der isolierten Verbindung(en) kann durch Techniken des Standes der Technik bestimmt werden. Diese umfassen Hochleistungs-Flüssigkeitschromatographie (HPLC), Gaschromatographie (GC), spektroskopische Verfahren, Färbeverfahren, Dünnschichtchromatographie, NIRS, Enzymtest oder mikrobiologische Tests. Diese Analyseverfahren sind zusammengefasst in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ullmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

Die Erfindung wird nun anhand der folgenden nicht-limitierenden Beispiele und unter Bezugnahme auf beiliegende Figuren näher beschrieben.

### EXPERIMENTELLER TEIL

### Fermentationsbeispiel 1:

Aus einer Stammkultur eines an sich bekannten DAP-Produktionsstammes (vgl. WO 2007/113127, Beispiel 2, Seite 14; worauf hiermit ausdrücklich Bezug genommen wird) (Lagerung bei -80°C) wurde eine Teilmenge Zellen entnommen, auf Festmedium (jeweilige Medienzusammensetzungen vgl. Tabelle 2) in einer Petrischale (Kultur 1) ausgestrichen und anschließend bei 30°C für 72 h inkubiert. Die so gewonnenen Zellen wurden in 0,9 %-iger NaCl-Lösung aufgenommen, erneut auf Festmedium in einer Petrischale (Kultur 2) ausgestrichen und anschließend bei 30°C für weitere 24 h inkubiert. Anschließend wurden die Zellen mit einer Impföse von der Petrischale (Kultur 2) in 200 mL eines 2L-Schüttelkolben mit 2 Schikanen (Zusammensetzung analog Platten- und Batchmedium) überimpft und bei 30°C auf einem Orbitalschüttler bei einer Drehzahl von 250 Upm für 24 h inkubiert.

Der Inhalt der Schüttelkolben diente als Vorkultur zur Inokulation eines 75 L-Fermenter mit einem Füllvolumen von 50 L. Der pH-Wert wurde mit Hilfe von Ammoniakgas auf pH 6,8 geregelt. Die Begasungsrate betrug ca. 0,33 vvm.

Die Hauptkultur wurde in einem 5 m³-Kessel mit 700 L Füllvolumen in der Batch-Phase durchgeführt. Hierzu wurde nach weiteren 24 h die Kultur aus dem 75 L-Fermenter in den 5 m³-Kessel überführt. Der pH-Wert wurde mit Ammoniak auf pH 6,8 geregelt. Der gelöste Sauerstoff wurde im Bereich von 20 bis 30 % (Luftsättigung) durch Anpassung der Begasungsrate und Rührerdrehzahl geregelt.

Nach ca. 24 h sank die Glucosekonzentration des Batch-Mediums auf einen Wert unter 1 g/L und die Zudosierung des Feeds wurde gestartet. Nach ca. 80 h wurde das Endvolumen der Fermentation von ca. 3200 L erreicht. Die Endkonzentrationen in der Fermentation betrugen OD₆₁₀: 140, 1,5-Diaminopentan: 72 g/L, Lysin x HCl: 15 g/L, Acetyldiaminopentan: 10 g/L.

Die Zellabtrennung erfolgte unter Verwendung einer Vollmantelzentrifuge, wobei die Zellen im Trüblauf um den Faktor 3,3 aufkonzentriert wurden und die Trübung im Klarlauf bei ca OD₆₁₀ 5 lag. Der Klarlauf wurde mit 50%iger NaOH auf pH 13,5 eingestellt.

**Tabelle 2: Zusammensetzungen der Medien**

| | | | Vorkultur | Hauptkultur | Hauptkultur |
|---|---|---|---|---|---|
| | Platte | Kolben | Batch | Batch | Feed |
| Agar | 20,00 g/L | | | | |
| Glucose Monohydrat | 62,48 g/L | 62,48 g/L | 18,76 g/L | 62,50 g/L | 431,79 g/L |
| Ammoniumsulfat | 24,29 g/L | 24,29 g/L | | 24,29 g/L | 130,00 g/L |
| Hefeextrakt, sprühgetrocknet | 15,16 g/L | 15,16 g/L | 18,94 g/L | 19,00 g/L | 17,40 g/L |
| Harnstoff | | | | | 13,79 g/L |
| Citronensäure Monohydrat | 2,04 g/L | 2,04 g/L | 2,04 g/L | 2,04 g/L | 1,55 g/L |
| Dinatriumhydrogenphosphat | 1,24 g/L | 1,24 g/L | | | |
| Magnesiumsulfat Heptahydrat | 1,25 g/L | 1,25 g/L | 1,25 g/L | 1,25 g/L | 1,13 g/L |
| Kaliumdihydrogenphosphat | 1,24 g/L | 1,24 g/L | 2,44 g/L | 2,48 g/L | 2.24 g/L |
| Calciumsulfat Dihydrat | 168,00 mg/L | 168,00 mg/L | 0,17 g/L | 0,17 g/L | 0,13 g/L |
| Eisensulfat Heptahydrat | 70,48 mg/L | 70,48 mg/L | 0,07 g/L | 0,07 g/L | 0,06 g/L |
| Zinksulfat Heptahydrat | 28,00 mg/L | 28,00 mg/L | 28,00 mg/L | 28,00 mg/L | 25,21 mg/L |
| Mangansulfat Monohydrat | 14,35 mg/L | 14,35 mg/L | 14,00 mg/L | 14,00 mg/L | 12,90 mg/L |
| Borsäure | 428,70 µg/L | 428,70 µg/L | 0,38 mg/L | 0,38 mg/L | 0,38 mg/L |
| Kupfersulfat Pentahydrat | 417,27 µg/L | 417,27 µg/L | 0,36 mg/L | 0,36 mg/L | 0,37 mg/L |
| Cobaltsulfat Heptahydrat | 337,24 µg/L | 337,24 µg/L | 0,30 mg/L | 0,30 mg/L | 0,30 mg/L |
| Nickelsulfat Hexahydrat | 284,37 µg/L | 284,37 µg/L | 0,24 mg/L | 0,24 mg/L | 0,25 mg/L |
| Dinatriummolybdat Dihydrat | 71,45 µg/L | 71,45 µg/L | 0,08 mg/L | 0,08 mg/L | 0,06 mg/L |
| Pantothensäure | 28,00 mg/L | 28,00 mg/L | 28,00 mg/L | 28,00 mg/L | 25,20 mg/L |
| Nicotinamid | 8,40 mg/L | 8,40 mg/L | 8,40 mg/L | 8,40 mg/L | 7,56 mg/L |
| Thiamin Hydrochlorid | 7,00 mg/L | 7,00 mg/L | 7,00 mg/L | 7,00 mg/L | 6,30 mg/L |
| Biotin | 4,20 mg/L | 4,20 mg/L | 4,20 mg/L | 4,20 mg/L | 3,78 mg/L |

Alle Medienbestandteile wurden bei 121°C für 30 min sterilisiert, mit Ausnahme der Vitamine, welche unter Verwendung eines 0,2 µm-Filters sterilfiltriert wurden.

### Ausführungsbeispiel 1: Gewinnung von DAP aus Fermentationsbrühe eines Lysin- und DAP-produzierenden Mikroorganismus

### a) Durchführung der thermischen Behandlung und Extraktion (Einzel-Batch)

In einen 1m³-Kessel aus Edelstahl wurden 750 kg (670 l) zellfreie, auf pH-Wert 13,5 mit NaOH eingestellte Fermentationsbrühe eingefüllt. Der Reaktorinhalt wurde auf Rückflusstemperatur (ca. 103°C) erhitzt und 5 Stunden lang refluxiert. Das dabei entstehende ammoniakhaltige Abgas wurde über einen Waschturm (mit Wasser als Waschflüssigkeit) abgefangen. Nach Abkühlen auf 60 °C wurden 140 kg n-Butanol zugefahren, 15 Minuten lang bei 60°C nachgerührt und der Ansatz 2 Stunden absitzen gelassen. Nach der Phasentrennung wurde die wässrige Unterphase in einen 1 m³-Behälter abgelassen. Die organische Phase wurde in einem weiteren Behälter gesammelt. Die wässrige Phase wurde nach Zugabe von je 35 L VE-Wasser bei 60°C weitere zwei Mal mit je 140 kg n-Butanol extrahiert.

Erhalten wurden insgesamt 590 kg organischer Extraktphasen, in denen 44,2 kg DAP enthalten waren (Gehalt ca. 7,5 %).

### b) Durchführung der Destillation:

In einem 1m³-Edelstahl-Kessel mit aufgesetzter Kolonne (4 theoretische Trennstufen) wurden 900 kg der vereinten organischen Phasen aus der Extraktion (Teilschritt a) vorgelegt. Unter Abdestillation von Wasser/n-BuOH wurden bei konstantem Volumen weitere 1400 kg Extraktlösung zugefahren. Anschließend wurde der Druck auf 200 mbar abgesenkt und destilliert, bis 360 kg Sumpf verblieben.

In einem weiteren Edelstahl-Kessel mit 400 L Volumen und aufgesetzter Kolonne mit 8 theoretischen Trennstufen wurde dieser Sumpf bei 40 mbar weiter destilliert. Nach Abtrennung eines BuOH-Vorlaufs und einer Mischfraktion wurden insgesamt 155,4 kg DAP mit einer durchschnittlichen Reinheit von 99,6 Gew.-% überdestilliert. Tetrahydropyridin als Nebenkomponente wurde in keiner Fraktion mit mehr als 0,4 % (GC-FL) gefunden.

### Testbeispiel 1: Untersuchung der Geschwindigkeit des Stoffübergangs bei der Extraktion

Die Einstellung des Verteilungsgleichgewichts eines erfindungsgemäß hergestellten organischen DAP-Extraktes wurde im 2,5-1-Reaktor unter intensivem Rühren bei 60 °C durch Entnahme einer zweiphasigen Probe zu verschiedenen Zeitpunkten über das Bodenventil und sofortige Trennung der Phasen nach Entmischung untersucht. Trotz einer gewissen Ungenauigkeit durch die erforderliche Absitzzeit der Probe kann geschlossen werden, dass die Verteilung sehr schnell erfolgt - die Probe nach 15 Sekunden wies 98,5 % des nach 5 Minuten gefundenen DAP auf. Eine Nachrührzeit von 15 Minuten ist somit als ausreichend anzusehen.

### Testbeispiel 2: Untersuchung der Phasentrennungsgeschwindigkeit

Die Geschwindigkeit der Phasentrennung wurde in einem 2,5-1-Doppelmantelreaktor mit 3-stufigem Balkenrührer und 4 Stromstörern untersucht.

Die Versuchsergebnisse für Ansätze (je 4 aufeinander folgende Extraktionen) mit und ohne thermische Behandlung sind in den Tabellen 3 und 4 zusammengefasst.

**Tabelle 3: Phasentrennzeiten bei Extraktion einer Brühe mit Hefeextrakt ohne thermische Behandlung**

| Extraktion (ohne Verkochung) | wässr. Phase | 200ml BuOH [g] | Rühren bei 400Upm [min] | Absitzzeit im Reaktor [min] | wässr. Phase [g] / [ml] | org. Phase org. Phase [g] / [ml] |
|---|---|---|---|---|---|---|
| 1 | 881,0 | 165,5 | 5 | 5 | 849,4/770 | 196,2/232 |
| 2 | 842,6 | 165,5 | 5 | 20 | 831,4/750 | 173,4/210 |
| 3 | 823,4 | 165,9 | 5 | 30 (noch 3mm Mulm) | 827,5/745 | 158,8/192 |
| 4 | 820,5 | 164,8 | 5 | 10 (Thermostat aus, Mulm)¹⁾ | 806,9/720 | 175,4/212 |

| | | | | | | |
|---|---|---|---|---|---|---|
| ¹⁾ Emulsion als Zwischenphase | | | | | | |

**Tabelle 4: Phasentrennzeiten bei Extraktion einer Brühe mit Hefeextrakt nach thermischer Behandlung (4h, 104 °C)**

| Extraktion (mit Verkochung) | wässr. Phase [g] | 188ml BuOH [g] | Rühren bei 400Upm [min] | Absitzzeit im Reaktor [min] | wässr. Phase [g ] / [ml] | org. Phase [g] / [ml] |
|---|---|---|---|---|---|---|
| 1 | 831,4 | 166,4 5 (200ml!) | | 2 | 817 / 740 | 170,8 / 204 |
| 2 | 810,1 | 155,6 | 5 | 2 | 779,3 / 700 | 182,4 / 224 |
| 3 | 772,1 | 155,6 | 5 | 2 | 770,1 / 690 | 156,2 / 193 |
| 4 | 763,1 | 155,6 | 5 3/4 | ∼2 | 766,3 / 780 | 149,7 / 186 |

### Testbeispiel 3: Verkochung und Acetyl-DAP-Spaltung

Es wurde beobachtet, dass der Produktionsorganismus einen Teil des gebildeten DAPs zusätzlich an einer der beiden Aminogruppen acetyliert.

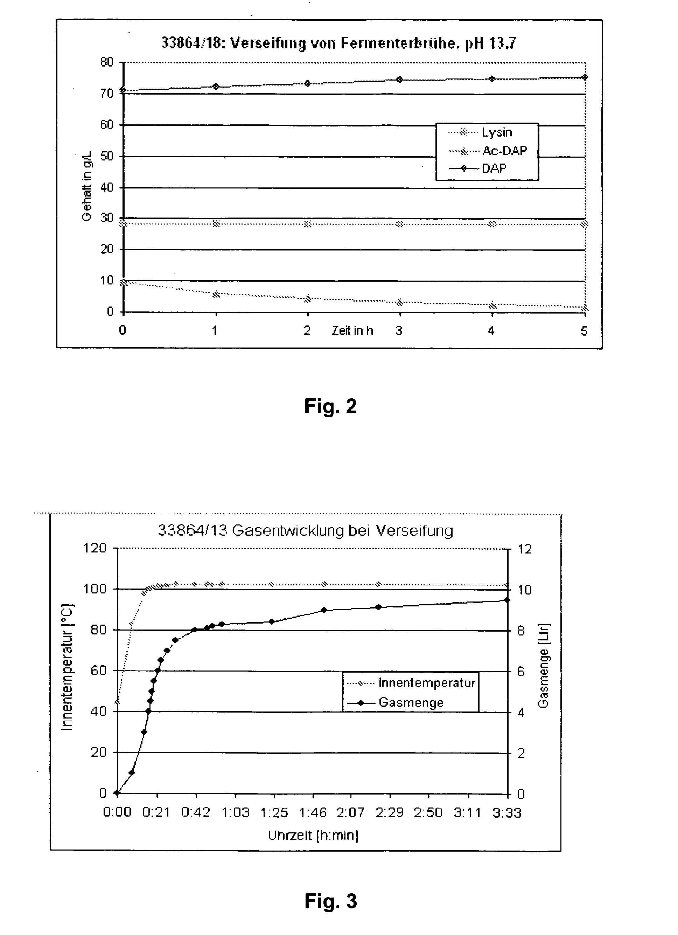

Es konnte gezeigt werden, dass sich das Acetyl-Diaminopentan durch Rückflusskochen der auf einen pH-Wert oberhalb von 13 alkalisch gestellten Fermentationsbrühe unter Freisetzung des Diaminopentans verseifen lässt (s. Fig. 2). Dadurch kann die Ausbeute gesteigert werden. Die Verseifung unter sauren Bedingungen (pH 1 mit H₂SO₄) verläuft dagegen nur sehr langsam.

Beim Rückflusskochen wird bereits bei ca. 95 °C beginnender Rückfluss beobachtet, im weiteren Verlauf stellt sich eine Rückflusstemperatur von ca. 103-105 °C im Sumpf ein. Bei der Verseifung wird vor allem während des Hochheizens die Freisetzung von Ammoniak beobachtet (s. Fig. 3).

Für die erfolgreiche Verkochung ist insbesondere ein pH-Wert von mindestens 13,5 von Vorteil.

### Testbeispiel 4: Abhängigkeit der Phasentrenngeschwindigkeit von der Dauer der thermischen Behandlung

Jeweils 300 mL einer erfindungsgemäß hergestellten Fermentationsbrühe mit pH 13,0 wurden in einem 0,75-L Doppelmantelreaktor mit Impellerrührer nach der thermischen Behandlung 3 Mal bei 60 °C nach Rühren mit 350 U/min mit jeweils 100 mL n-BuOH (wassergesättigt) extrahiert. Die Ergebnisse sind in Tabelle 5 zusammengefasst.

**Tabelle 5: Absitzzeit nach verschiedener Dauer der thermischen Behandlung**

| Dauer der therm. Behandlung [h] | Absitzzeit im Reaktor [min] bei Extraktion 1/2/3 |
|---|---|
| Ohne | 4 / 3(Mulm) / 3 (Mulm) |
| 0,5 | 3/4/5 |
| 1 | 2/3/3 |
| 2 | 2/3/3 |
| 4 | 2/2/3 |

### Testbeispiel 5: pH-Abhängigkeit des Verteilungskoeffizienten

Je 2 kg einer erfindungsgemäß hergestellten Fermentationsbrühe wurden mit DAP auf einen Gehalt von 10 % aufgestockt und der pH durch Zugabe von NaOH auf 11,0, 12,0 bzw. 13,5 eingestellt. Bei 60°C wurde zur Bestimmung der Verteilungskoeffizienten jeweils fünf mal mit 150 g wassergesättigtem n-BuOH extrahiert. Die analytisch bestimmten DAP-Gehalte der wässrigen und organischen Phasen und die Verteilungskoeffizienten sind in Tabelle 6 angegeben.

**Tabelle 6: Verteilung des DAP zwischen Wasser und n-BuOH bei verschiedenen pH-Werten**

| pH | | 13,5 | | | | |
|---|---|---|---|---|---|---|
| Exktraktion | | 1 | 2 | 3 | 4 | 5 |
| Wässrige | | | | | | |
| Phase | [%] | 10,19% | 7,42% | 4,85% | 3,05% | 1,86% |
| Org. Phase | [%] | 11,12% | 19,08% | 17,85% | 14,35% | 10,92% |
| K | | 1,09 | 2,57 | 3,68 | 4,71 | 5,87 |
| | | | | | | |
| pH | | 12,0 | | | | |
| Exktraktion | | 1 | 2 | 3 | 4 | 5 |
| Wässrige Phase | [%] | 7,93% | 5,44% | 3,76% | 2,67% | 1,94% |
| Org. Phase | [%] | 7,40% | 14,40% | 12,90% | 10,20% | 7,40% |
| K | | 0,93 | 2,65 | 3,43 | 3,81 | 3,81 |
| | | | | | | |
| pH | | 11,0 | | | | |
| Exktraktion | | 1 | 2 | 3 | 4 | 5 |
| Wässrige Phase | [%] | 8,69% | 8,12% | 7,81% | 7,49% | 7,29% |
| Org. Phase | [%] | 7,20% | 7% | 6,50% | 5,10% | 4,30% |
| K | | 0,83 | 0,86 | 0,83 | 0,68 | 0,59 |

### Testbeispiel 6: Abhängigkeit der Spaltungskinetik von AcDAP vom pH-Wert

In einem 0,75L-Doppelmantelreaktor wurden je 500g erfindungsgemäß hergestellter Fermentationsbrühe mit hohem Gehalt an AcDAP durch Zugabe von 50%iger NaOH auf den gewünschten pH eingestellt und auf Rückflusstemperatur erhitzt. Nach 0,5, 1, 2 und 4 h wurden Proben gezogen und per quantitativer HPLC vermessen. Die Ergebnisse sind in Tabelle 7 zusammengefasst.

**Tabelle 7: Abhängigkeit der Spaltungsgeschwindigkeit von AcDAP vom pH-Wert**

| Verkochung [h] | pH | Lysin | Ac-DAP | DAP | pH | Lysin | Ac-DAP | DAP | pH | Lysin | Ac-DAP | DAP |
|---|---|---|---|---|---|---|---|---|---|---|---|---|
| | | jeweils [Gew%] | | | | jeweils [Gew%] | | | | jeweils [Gew%] | | |
| 0 | 12,99 | 0 | 2,131 | 4,405 | 13,58 | 0 | 2,027 | 4,426 | 13,98 | 0 | 1,903 | 4,051 |
| 0,5 | 13,02 | 0 | 2,011 | 4,568 | 13,52 | 0 | 1,56 | 4,877 | 13,90 | 0 | 0,439 | 5,013 |
| 1 | 12,92 | 0 | 1,908 | 4,652 | 13,48 | 0 | 1,269 | 5,089 | 13,90 | 0 | 0,145 | 5,21 |
| 2 | 12,81 | 0 | 1,791 | 4,73 | 13,42 | 0 | 0,9 | 5,363 | 13,88 | 0 | 0 | 5,327 |
| 4 | 12,61 | 0 | 1,542 | 4,827 | 13,34 | 0 | 0,508 | 5,622 | 13,88 | 0 | 0 | 5,434 |

### Testbeispiel 7: Salzfällung

Zu 130 g einer erfindungsgemäß hergestellten Extraktphase mit einem DAP-Gehalt von ca. 4.1 Gew.-% wurde bei 25-30 °C eine Lösung von 7,2 g Adipinsäure in 70 g wassergesättigtem Butanol zugetropft. Das Reaktionsgemisch wurde auf 3 °C abgekühlt, der Feststoff abfiltriert und über Nacht im Stickstoffstrom getrocknet. Erhalten wurden 12,7 g eines weißen Feststoffs, der per ¹³C-NMR als 1:1-Mischung von Adipinsäure und DAP charakterisiert wurde.

## Patentansprüche

1. Verfahren zur Isolierung von 1,5-Diaminopentan (DAP) aus einer DAP-haltigen Fermentationsbrühe, wobei man die Fermentationsbrühe a) auf einen pH-Wert von >11 einstellt, b) thermisch behandelt, wobei man die thermische Behandlung unter Bedingungen durchführt, die eine hydrolytische Spaltung von gegebenenfalls vorhandenem Acetyl-DAP bewirken, c) DAP mit einem organischen Extraktionsmittel extrahiert, und d) DAP aus der abgetrennten organischen Phase isoliert.

2. Verfahren nach Anspruch 1, wobei man die alkalisierte Fermentationsbrühe thermisch behandelt, indem man auf Rückflusstemperatur erhitzt.

3. Verfahren nach einem der vorhergehenden Ansprüche, wobei man die DAP-Extraktion mit einem dipolar-protischen organischen Lösungsmittel durchführt.

4. Verfahren nach einem der vorhergehenden Ansprüche, wobei man aus der Fermentationsbrühe vor der Alkalisierung zelluläre Bestandteile entfernt.

5. Verfahren nach einem der vorhergehenden Ansprüche, wobei man die DAPhaltige Phase des Extraktionsschrittes destillativ aufreinigt oder DAP daraus ausfällt.

6. Verfahren zur fermentativen Erstellung von DAP, wobei man einen Lysin-produzierenden Mikroorganismus unter Lysin- und gegebenenfalls DAP-produzierenden Bedingungen kultiviert und das gebildete DAP unter Anwendung eines Verfahrens nach einem der Ansprüche 1 bis 5 isoliert.

7. Verfahren nach Anspruch 6, wobei der Lysin-produzierende Mikroorganismus Lysin Decarboxylase-Aktivität enthält.

8. Verfahren nach Anspruch 6, wobei man die Lysin-haltige Fermentationsbrühe mit gegebenenfalls immobilisierter Lysin-Decarboxylase in Kontakt bringt, um Lysin zu DAP zu decarboxylieren.

9. Verfahren zur Herstellung eines DAP-haltigen Polymers, wobei man zunächst monomeres DAP nach einem Verfahren gemäß einem der Ansprüche 1 bis 5
fermentativ herstellt und isoliert und zusammen mit wenigsten einem weiteren Comonomer polymerisiert

10. Verfahren nach Anspruch 9, wobei das Comonomer ausgewählt ist unter Polyisocyanaten und Polycarbonsäuren, sowie deren Salzen, Estern und Anhydriden.

11. Verfahren nach Anspruch 9, wobei man wenigstens ein Comonomer dem isolierten DAP zusetzt oder ein Gemisch aus DAP und wenigstens einem Comonomer aus einer DAP-Fällung einsetzt.

12. Verfahren nach Anspruch 11, wobei das DAP/Comonomer-Gemisch aus einer Salzfällung nach Anspruch 5 resultiert.

13. Verfahren nach Anspruch 12, wobei das Comonomer eine Polycarbonsäure ist.

## Claims

1. A process for isolating 1,5-diaminopentane (DAP) from a DAP-comprising fermentation broth, wherein a) the fermentation broth is adjusted to pH >11, b) the fermentation broth is thermally treated, wherein thermal treatment is carried out under conditions which cause optionally present acetyl-DAP to be cleaved hydrolytically, c) DAP is extracted with an organic extractant, and d) DAP is isolated from the removed organic phase.

2. The process according to claim 1, wherein the alkalized fermentation broth is thermally treated by heating to reflux temperature.

3. The process according to any of the preceding claims, wherein DAP is extracted with a dipolar protic organic solvent.

4. The process according to any of the preceding claims, wherein cellular components are removed from the fermentation broth prior to alkalization.

5. The process according to any of the preceding claims, wherein the DAP-comprising phase of the extraction step is purified by distillation or DAP is precipitated wherefrom.

6. A process for the fermentative production of DAP, wherein a lysine-producing microorganism is cultured under lysine-producing and, if appropriate, DAP-producing conditions and the DAP formed is isolated by applying a process according to any of claims 1 to 5.

7. The process according to claim 6, wherein the lysine-producing microorganism comprises lysine decarboxylase activity.

8. The process according to claim 6, wherein the lysine-comprising fermentation broth is contacted with optionally immobilized lysine decarboxylase in order to decarboxylate lysine to give DAP.

9. A process for preparing a DAP-comprising polymer, wherein DAP monomer is first fermentatively produced and isolated by a process according to any of claims 1 to 5 and then polymerized together with at least one further comonomer.

10. The process according to claim 9, wherein the comonomer is selected from among polyisocyanates and polycarboxylic acids, and salts, esters and anhydrides thereof.

11. The process according to claim 9, wherein at least one comonomer is added to the isolated DAP, or a mixture of DAP and at least one comonomer from a DAP precipitation is employed.

12. The process according to claim 11, wherein the DAP/comonomer mixture is produced by salt precipitation according to claim 5.

13. The process according to claim 12, wherein the comonomer is a polycarboxylic acid.

## Revendications

1. Procédé pour isoler le 1,5-diaminopentane (DAP) à partir d'un bouillon de fermentation contenant du DAP, dans lequel a) on ajuste le pH du bouillon de fermentation à une valeur supérieure à 11, b) on le soumet à un traitement thermique, en mettant en oeuvre le traitement thermique dans des conditions qui conduisent à une dissociation hydrolytique de l'acétyl-DAP éventuellement présent, c) on extrait le DAP à l'aide d'un agent d'extraction organique, et d) on isole le DAP de la phase organique séparée.

2. Procédé selon la revendication 1, dans lequel on soumet à un traitement thermique le bouillon de fermentation alcalinisé, par chauffage à la température de reflux.

3. Procédé selon l'une des revendications précédentes, dans lequel on met en oeuvre l'extraction du DAP à l'aide d'un solvant organique dipolaire-protique.

4. Procédé selon l'une des revendications précédentes, dans lequel, avant l'alcalinisation, on élimine les constituants cellulaires du bouillon de fermentation.

5. Procédé selon l'une des revendications précédentes, dans lequel on purifie par distillation la phase contenant du DAP de l'étape d'extraction, ou on en isole le DAP par précipitation.

6. Procédé de préparation fermentative de DAP, dans lequel on cultive un micro-organisme produisant la lysine, dans des conditions de production de lysine et éventuellement de DAP, et, par utilisation d'un procédé selon l'une des revendications 1 à 5, on isole le DAP formé.

7. Procédé selon la revendication 6, dans lequel le micro-organisme produisant la lysine contient une activité de lysine décarboxylase.

8. Procédé selon la revendication 6, dans lequel on met le bouillon de fermentation contenant la lysine en contact avec de la lysine décarboxylase éventuellement immobilisée, pour décarboxyler la lysine en DAP.

9. Procédé de préparation d'un polymère contenant du DAP, dans lequel on prépare par fermentation et on isole d'abord le DAP monomère par un procédé selon l'une des revendications 1 à 5, et on le polymérise en même temps qu'au moins un autre comonomère.

10. Procédé selon la revendication 9, dans lequel le comonomère est choisi parmi les polyisocyanates et les poly(acides carboxyliques), ainsi que leurs sels, esters et anhydrides.

11. Procédé selon la revendication 9, dans lequel on ajoute au DAP isolé au moins un comonomère, ou on utilise un mélange de DAP et d'au moins un comonomère provenant de la précipitation du DAP.

12. Procédé selon la revendication 11, dans lequel le mélange DAP/comonomère est obtenu à partir d'une précipitation de sels selon la revendication 5.

13. Procédé selon la revendication 12, dans lequel le comonomère est un poly(acide carboxylique).
